# EUROPEAN PATENT APPLICATION

(11) **EP 4 472 090 A2**
(43) Date of publication of application: **04.12.2024**
(21) Application number: 24207502.6
(22) Date of filing: 16.12.2020
(51) Int. Cl.: H04B 5/72

(54) **PRE-FILLED SYRINGE WITH INJECTION END POINT SIGNALLING DEVICE**

(62) Divisional of application: 20853602.9
(71) Applicant: Biocorp Production S.A.S., 63500 lssoire (FR)
(72) Inventor: MARCOZ, Alain, 63160 MONTMORIN (FR)
(74) Representative: Thurgood, Alexander John

(57) **Abstract**

Pre-filled syringe comprising a needle shroud in which the shroud translates from a first, retracted position, to a second, extended position, and comprising an injection endpoint signalling device mounted thereon. The injection endpoint signalling device comprises a near field communications (NFC) circuit and an activation switch, and in the first, shroud-retracted position, the activation switch maintains the NFC circuit in an inactive state in which an injection endpoint information is inaccessible to the NFC circuit, and in the second, shroud-extended position, the activation switch maintains the NFC circuit in an active state in which the injection endpoint information is accessible to the NFC circuit. The activation switch is moved from the inactive state to the active state via mutually cooperating surface engagement between a part of the shroud and the activation switch.

## Description

The present invention relates to pre-filled syringes and associated technology. In particular, the present invention relates to a signalling assembly for pre-filled syringes using a near field communications circuit, commonly abbreviated as NFC.

Pre-filled syringes are known per se to the skilled person and are in common use for the administration of a variety of fixed or unit doses of substances, be they medicaments or other substances. For example, pre-filled syringes are commonly used for the administration of drugs such as vaccines for immunisation campaigns and programmes, or for the treatment of long-term pathologies, such as, for example, diabetes, or other disorders which require management with administration of fixed, pre-measured and stored doses of medicaments, for example, anti-venoms used in the treatment of snake or spider bites, or for emergency injections for the treatment or onset of other potentially life-threatening situations, such as acute pain or trauma, myocardial infarct, anaphylaxis, bacterial or toxic shock and the like. The applications for pre-filled syringes are thus widespread and well known.

Such pre-filled syringes generally comprise:
an elongated hollow syringe body having a proximal extremity and a distal extremity, with a first opening at the proximal extremity and a collar, or flange, projecting outwardly of the hollow syringe body at said proximal extremity around said first opening;
an injection needle mounted, or mountable, at the distal extremity of the hollow elongated syringe body and closing a second opening of the hollow elongated syringe body at said distal extremity;
a controlled amount of injectable material introduced into the hollow body; and
a plunger configured and dimensioned to be inserted into said hollow elongated syringe body via the proximal extremity and corresponding proximal opening of the hollow syringe body, the plunger having a plunger body comprising a stopper located at a distal extremity of the plunger body, and a plunger head located at a proximal extremity of said plunger body.

One of the general problems with such pre-filled syringes is being able to tell when the syringe has actually been used, in order to avoid attempted re-use, or alternatively, for tracking purposes, for example in order to know whether and how much of the injectable substance has been administered from the pre-filled syringe. To this end, various tracking systems have been associated with such pre-filled syringes in order to attempt to overcome this general problem.

For example, international patent application published as WO2014089086 relates to a method for using an electronic medicament device such as an auto-injector including a medication such as epinephrine for treating anaphylactic shock. The device includes a sensor, an ID tag, such as a RFID, NFC, or other tag for short range wireless communications, such as Bluetooth communications, a memory, a display, and a speaker, as well as a processor and communication interfaces, the processor interconnecting one or more of the components, and the communication interface including an interface for communication via wifi, a mobile carrier network, or satellite. The processor is configured to communicate with at least one remote system such as a mobile phone via the communication interface in response to the occurrence of an event, such as the administration of the medication and expiration of the medication. The sensor detects activation of the device, and includes a frangible element that completes or breaks an electronic circuit when the device is activated. The sensor provides a signal to the ID tag to perform an action in response to use of the auto-injector device, and alters the memory to indicate that the the device is used, along with a log of the time of use. The ID tag also provides information from the auto-injector device to a wireless reader such as a NFC-enabled mobile device, e.g. a mobile phone. The mobile phone reads medicament information that is either printed on the auto-injector device or stored in the auto-injector device memory using RFID, NFC, or other wireless communication.

Similarly, US patent application published as US2019038840 discloses a pre-filled syringe comprising a complex arrangement of two antennae, a first, transmission antenna, configured to transmit a control signal to an external device, control electronics connected to the transmission first antenna configured to provide instructions to the transmission antenna to transmit the control signal, and a second, bypass antenna positioned and configured to prevent the control electronics from providing the instructions to the transmission antenna when the bypass antenna is in an undisturbed position and to permit the control electronics to provide the instructions to the transmission antenna when the bypass antenna is displaced from the undisturbed position. The complex arrangement of the two antennae and control electronics is integrated at a proximal end of the syringe plunger and is covered by a press button. The bypass antenna is configured as a physically destructible electric switch, for which electrical contact is broken when the press-button is depressed by the user of the syringe. Depressing the press button causes the electrical contact to the bypass antenna to be destroyed irreparably, activating the primary antenna circuit and signalling the beginning of use of the syringe.

Furthermore, an international patent application published as WO2018111969A1 discloses a plunger rod adapted to push a medication out of a syringe comprising a shaft sized and dimensioned to act on a piston on the syringe and a finger actuated head portion containing at least two subunits of a wireless sensor. In a pre-activation configuration, the subunits are spaced apart from each other by a physical barrier and the wireless sensor is non-operational. In a post-activation configuration, the subunits are connected to each other and the sensor is operational to send a signal. A user activates the finger actuated head portion to reversibly move the physical barrier and moves the plunger rod from the pre-activation configuration to the post-activation configuration. The signal sent by the sensor comprises information relating to ejecting the medication out of the syringe, and is received by a remote receiver.

Despite the solutions disclosed in the abovementioned documents, there still remain a number of challenges to injection endpoint detection and signalling using near field communication to be overcome. This is particularly so for pre-filled syringes that are specifically modified to function in a particular way. For example, prefilled syringes that integrate safety mechanisms to protect a user against needle-stick injury once injection has been completed are also known. Such pre-filled syringes often comprise a system that automatically protects the user from, or removes, the needle after injection has been completed.

According therefore to one object, the applicant provides an injection endpoint signalling device adapted and configured for mounting on, and use with, a pre-filled syringe comprising a needle safety mechanism, in which information relating to the injection endpoint is only capable of being signalled via a wireless communications circuit such as a near field communications circuit, once the needle safety mechanism has been activated.

These and other objects will be described hereinafter or become apparent from the following specification.

Accordingly, one object of the present is an injection endpoint signalling device, adapted and configured to be mounted onto a pre-filled syringe, the pre-filled syringe comprising a post-injection needle shroud, the needle shroud being configured to translate from a first position in which the shroud is retracted and the needle of the pre-filled syringe is exposed, to a second position in which the shroud is extended and the needle of the pre-filled syringe is completely surrounded by the shroud, wherein:
the injection endpoint signalling device comprises a wireless injection endpoint signalling system comprising a near field communications (NFC) circuit and an activation switch; and
when mounted on the pre-filled syringe:
   in the first, shroud-retracted position, the activation switch maintains the NFC circuit in an inactive state in which said NFC circuit is off, and in which an injection endpoint information is inaccessible to the NFC circuit; and
   in the second, shroud-extended position, the activation switch maintains the NFC circuit in an active state in which said NFC circuit is on, and in which the injection endpoint information is accessible to the NFC circuit;
wherein the activation switch is moved from the inactive state to the active state via mutually cooperating surface engagement between a part of the shroud and the activation switch.

It is to be understood from the present that the pre-filled syringe is substantially as described generally above and comprises:
an elongated hollow syringe body having a proximal extremity and a distal extremity, with a first opening at the proximal extremity and a collar, or flange, projecting outwardly of the hollow syringe body at said proximal extremity around said first opening;
an injection needle mounted, or mountable, at the distal extremity of the hollow elongated syringe body and closing a second opening of the hollow elongated syringe body at said distal extremity;
a controlled amount of injectable material introduced into the hollow body; and
a plunger configured and dimensioned to be inserted into said hollow elongated syringe body via the proximal extremity and corresponding proximal opening of the hollow syringe body, the plunger having a plunger body comprising a stopper located at a distal extremity of the plunger body, and a plunger head located at a proximal extremity of said plunger body.

Generally, most such pre-filled syringes are only intended for single use, for example, when administering vaccines or other single dose administration medicines, after which use they should be disposed of according to current appropriate good usage practice.

The pre-filled syringe comprises a post-injection needle shroud. By the expression "post-injection needle shroud", this is to be understood, for the purposes of the present specification, as referring to pre-filled syringes that are equipped with either a passive or an active needle shroud which is operational on completion of, or immediately thereafter, ejection of the substance contained within the syringe. In this context, the expressions "passive" and "active" with regard to a needle safety shroud refer respectively:
"passive" - needle shrouds that are activated automatically as a result of movement of one or more components of the pre-filled syringe with regard to the needle shroud, the activation being independent of any action by the user of the pre-filled syringe other than effecting an ejection of substance from the syringe chamber;
"active" - needle shrouds that are activated via a separate, or deliberate operation to the normal expelling of injectable substance from the syringe chamber, and effected by user of the pre-filled syringe at the end of the expelling operation.

As mentioned above, the needle shroud is configured to translate from a first position in which the shroud is retracted and the needle of the pre-filled syringe is exposed, to a second position in which the shroud is extended and the needle of the pre-filled syringe is completely surrounded by the shroud. An example of a pre-filled syringe that operates in this manner is available under the tradename "BD Ultrasafe Passive" ^{™}, sold by Becton Dickinson, and which relates to a passive needle guard mechanism intended for pre-filled ISO standard glass syringes, for which injection can be carried out with one hand. In this device the needle safety shroud is in a first position in which the needle is exposed, at the beginning and all through the injection movement as the plunger is moved in a distal direction along a central longitudinal axis of the syringe, until the plunger head of the syringe engages with a pair of elastically deformable release wings which are thereby pushed radially outwardly from the central axis, and which in turn are brought to bear on elastically deformable portion of the needle shroud at a proximal end of the needle shroud. The elastic deformation of the needle shroud at the proximal end of the shroud causes the bore of the shroud to widen slightly along its length. An abutment sleeve is located within the bore of the shroud and is positioned in fixed axial engaging contact with the outside surface of the syringe barrel. A pre-constrained biasing spring is located in the bore of the shroud between the distal end of the shroud and a distally facing contact surface of the abutment sleeve, the spring abutting against the distally facing contact surface of the abutment sleeve. The elastically deformed widening of the shroud caused by the plunger head interacting with the elastically deformable wings on completion of an injection allows the pre-constrained biasing spring to expand axially along the central axis and at the same time push against the distally facing contact surface of the abutment sleeve, thereby driving the shroud in a distal direction. As the shroud is moved in the distal direction, from the shroud retracted position towards the shroud extend position, the shroud begins to cover the needle which was exposed during injection.

Furthermore, as the shroud moves in a distal direction, the elastic deformation of the proximal end of the shroud is released and the internal diameter of the bore of the shroud begins to return to normal. The shroud is prevented from extending distally beyond a predetermined axial limit by at least one recess located on the shroud adjacent a proximal end of the shroud, which recess receives at least one radially projecting spur located on and extending from the abutment sleeve. The position of the proximal recess, the abutment sleeve, and the corresponding expansion of the biasing spring determine the extent of axial travel of the shroud from the first, needle exposed position, to the second, needle shrouded position, and respectively, shroud extended position.

As mentioned above, the injection endpoint signalling device also comprises a wireless injection endpoint signalling system comprising a near field communications circuit and an activation switch. When the injection endpoint signalling device is mounted on a pre-filled syringe such as the one described above, in the first, shroud-retracted position, the activation switch maintains the near field communications circuit in an inactive state in which said circuit is off, and in which an injection endpoint information is inaccessible to the NFC circuit. As used herein, the terms "inactive" and "off" relate to the impossibility for the NFC circuit to retrieve or discover the injection endpoint information. The endpoint information in question can for example be as simple as a single bit of data, or an electrical pulse, or the simple state of an open or closed electrically conducting or semi-conducting gate allowing the passage of charged particles, such as electrons.

Conversely, in the second, shroud-extended position, the activation switch maintains the NFC circuit in an active state in which said circuit is on, and consequently in which the injection endpoint information is accessible to the NFC circuit. As used herein therefore, the terms "active" and "on" relate to the possibility for the NFC circuit to retrieve or discover the injection endpoint information, when such a circuit is energized in a known manner.

In this way, it is ensured that the endpoint information is only available to the NFC circuit when the activation switch is in the active, or "on" state, and that only injections which have actually been completed can be communicated via the NFC circuit to a separate NFC reader or suitably NFC-equipped smartphone device.

Near field communication (NFC) technology as a derivative or evolution of RFID technology is well known to the skilled person. It is described in detail in the international standards ISO/IEC 14443 and ISO/IEC 18000-3, with the former defining the functioning of ID cards used to store information, such as is found in NFC ID tags and the latter defining RFID communication used by NFC equipped devices. The basis of NFC is to be found in radio-frequency identification, or RFID, technology, which provides for suitably equipped hardware to both supply power to and communicate with an otherwise unpowered, or unenergized, and passive electronic tag using radio waves. Accordingly, the NFC circuit used in the present invention comprises a passive ID tag, which stores a set of information, such as for example, the type of injectable substance, the unit dose, concentration, expiry date, and the like, and any other useful or required information that can be appropriately stored within the limits of such a NFC ID tag. The NFC circuit also comprises suitable and corresponding communication components which would normally enable the NFC circuit, when energized, to exchange said information with another NFC enabled device, such as a smartphone. An antenna forming part of the NFC circuit is also provided, to capture radio waves of the given functional frequency of the NFC protocol and thereby energize the circuit.

Additionally, and as mentioned above, the activation switch of the near field communications circuit, when mounted on a pre-filled syringe as described above, is moved from the inactive state to the active state via mutually cooperating surface engagement between a part of the shroud and the activation switch. Accordingly, the activation switch engages with, and is moved by, a physical interaction with a part of the safety shroud mechanism.

Advantageously, and according to another object, the mutually cooperating surface engagement between the activation switch and corresponding part of the needle safety shroud is only provided when the shroud is positioned in a fully extended position. In other words, the near field communications circuit is only active when the shroud has reached its final, extended position, completely covering the needle of the pre-filled syringe. In this way, one can be certain that not only the pre-filled syringe is safe to dispose of, but additionally that the ejection and/or injection endpoint has been reached, and that the endpoint information is accordingly made accessible to the NFC circuit integrated into the endpoint signalling device, which endpoint information can then be signalled to a NFC-equipped reader device, through energization of the NFC circuit located in the injection point signalling device, in the usual manner.

According to yet another object, the activation switch is moved from the inactive state to the active state by cooperating surface engagement between a proximal part of the shroud and the activation switch. A suitable proximal part of the shroud can be the recess located in the shroud near the proximal end of the shroud, which recess receives the at least one radially projecting spur located on and extending from the abutment sleeve, as described above. In such a configuration, the activation switch is positioned, when mounted on the shroud, and in the inactive state, to occupy the space provided by the recess from a position above the recess facing into the bore of the shroud. When injection is complete, the shroud is moved axially in a proximal direction as described above due to the interaction of the biasing spring against the abutment sleeve, such that the projecting spur of the abutment sleeve engages in the recess of the shroud and pushes against the activation switch to move said switch, from an inactive or "off" state to an active, or "on" state.

According to a further object, the activation switch, when the circuit holder body is mounted on the pre-filled syringe body and/or the needle safety shroud, is located in parallel to, and along, the central longitudinal axis.

According to a still further object therefore, the activation switch is a displaceable, or movable, electrical contact.

According to another object, the displaceable, or movable, electrical contact is selected from the group consisting of a microswitch, a biased, or constrained, electrically conducting metal strip, and a movable electrically conducting surface. The displaceable, or movable, electrical contact is generally arranged to be movable or displaced, from the first, inactive, or "off" position in which no electric current or charge may pass through the circuit with which the electrical contact interacts, to a second, active, or "on" position in which an electrical contact is made allowing electrical charge or current to flow through the circuit with which the electrical contact interacts. In the case where an electrically conducting surface is implemented as the switch, such an electrically conducting surface can usefully comprise a conducting material distributed in, or on such a surface, for example by any of a range of techniques known to the skilled person, such as layering, embedding, deposition whether chemical or physical, etching, engraving, doping, and the like. In a particularly advantageous embodiment, the electrically conducting surface located on the electrical contact applicator comprises carbon or metal particles. This electrically conducting surface will form the electrical contact once the projecting spur of the abutment sleeve is caused to be recessed in the corresponding recess provided on the shroud when the shroud moves into the fully extended position. Until such position is attained, the electrically conducting surface is configured and organised to prevent the establishment of any electrical contact, thereby rendering the endpoint information accessible to the NFC circuit.

According to yet another object, the injection endpoint signalling device comprises a near field communications circuit holder body, wherein the circuit holder body is mounted on an outward facing surface of a longitudinal body of the pre-filled syringe.

In a still further object, and advantageously, the circuit holder body is mounted on an outward facing surface of the needle safety shroud. By "outward facing", it is to be understood that this refers to an outer surface of the shroud, ie. a surface which faces outwards as opposed to an inner, or inwardly facing surface of the shroud, which would face inwards into the bore of the shroud.

According to yet another object, the circuit holder body is mounted on the pre-filled syringe in a plane that lies parallel to the central longitudinal axis.

Additionally, and advantageously according to another object, the circuit holder body is mounted on the outwards facing surface of the needle shroud, and also engages with at least a part of the of the pre-filled syringe, such that the circuit holder body can not be removed from the shroud, but enables the shroud to be moved axially along the central longitudinal axis from the first, shroud retracted position, to the second, shroud extended position. The net result of such mounting is that the circuit holder body lies not only in parallel to the central longitudinal axis of the syringe, but also extends substantially orthogonal to, and either side of, said central longitudinal axis in said parallel longitudinal plane.

Furthermore, according to yet another object, and advantageously, the displaceable, or movable, electrical contact of the activation switch of the NFC circuit establishes an active electrical contact via translational movement of the shroud, in a direction parallel to the central longitudinal axis, from the first, inactive position in which no electrical contact is established, to the second, contact position establishing an electrical contact. The shroud thus serves to move, either directly, or indirectly, for example via the projecting spur of the abutment sleeve, the activation switch from an electrically gapped or electrically isolated area of the NFC circuit, thereby closing the circuit, and allowing current or charge to flow, and thereby also making the endpoint information accessible to the NFC circuit.

According to another object, the circuit holder body comprises a socket configured and dimensioned to receive and locate a NFC microcontroller of the near field communications circuit. The socket provided in the circuit holder body serves to prevent the NFC microcontroller from moving in relation to the circuit body holder, for example, when mounting the injection endpoint signalling device on the pre-filled syringe.

Additionally, and according to a further object, the near field communications circuit is advantageously integrated into a disk-shaped circuit board, the NFC microcontroller being located on a first face of the circuit board, and the activation switch being located on an opposite, second face of the circuit board. Such a configuration enables the disk-shaped circuit board to be seated, on the one hand, via the physical surface interaction between the microcontroller and the seating socket of circuit body holder, and, on the other hand, leaves the activation switch free for corresponding cooperating surface engagement with the needle shroud.

According to yet another object, the first face of the disk-shaped circuit board is held against an inward facing surface of a disk-shaped base of the circuit holder body by at least one or more retaining lugs. The retaining lugs help to hold the disk-shaped circuit board in the circuit holder body, and along with the socket, position the circuit board appropriately, and therefore the corresponding activation switch with respect to the needle shroud when the device is mounted on the pre-filled syringe.

In a further object, the retaining lugs are radially distributed about an axis of rotation of the disk-shaped base of the circuit holder body.

According to yet another object, the axis of rotation of the disk-shaped base of the circuit holder body lies perpendicular to the central longitudinal axis of the pre-filled syringe. From this it will be understood, and as mentioned elsewhere in the present specification, that the endpoint signalling device is at least in part disk-shaped, and that the disk when mounted on the pre-filled syringe lies in a plane which is both parallel, and orthogonal within the parallel plane, to the central longitudinal axis of the syringe. Accordingly, the axis of rotation of the disk-shaped base of the circuit holder body lies perpendicular to the horizontal plane which is parallel to the central longitudinal axis.

According to another object, the circuit holder body comprises at least one or more walls located on a periphery of, and extending in the same direction away from, the disk shaped base. The walls are shaped and configured to engage with at least part of the pre-filled syringe and/or needle shroud.

According therefore to a further object, the at least one or more extending walls are arc shaped and, when the device is mounted on the pre-filled syringe and/or the needle safety shroud, said walls engage in elastically deformable abutment with at least one side wall of the pre-filled syringe and/or the needle safety shroud.

It will thus be understood from the above that the circuit holder body advantageously comprises a base, preferably disk-shaped, and that this base is provided with, for example, a pair of walls extending from a periphery of said base, and away from said base, preferably in direction that is orthogonal to the plane of the base of the circuit holder body. Furthermore, the walls advantageously extend in a direction substantially parallel to the axis of rotation of the base of the circuit holder body to form an engagement surface that is elastically deformable when mounted on the pre-filled syringe and/or needle shroud, and which prevent any lateral movement of the injection endpoint signalling device about the central longitudinal axis.

According to yet another object, the at least one or more extending walls each comprise a prehensile shoulder, extending substantially orthogonal to, and away from, the central longitudinal axis from a proximal end of each of the respective extending walls. The prehensile shoulders are designed to engage and abut with a finger stop, otherwise known as a backstop, mounted on the needle shroud, and the shoulders provide a surface on which the fingers of one hand are brought to bear during use, whilst the plunger is pressed generally by a thumb of the same hand.

According to a further object, the prehensile shoulder extends, from a radially distant end of the shoulder, in a proximal direction to form a curled lip, configured to engage in elastically deformable clasping engagement with a corresponding finger-stop or backstop which extends orthogonally outwardly from the shroud. The curled lip serves to clamp or clasp the circuit body holder onto the backstop of the syringe, and prevent any undesired movement of the endpoint signalling device when the user is pressing the plunger with its thumb during injection.

According to yet another object, the prehensile shoulder is provided with one or more elastically deformable seating lugs, which extend proximally away from the shoulder, to assist in engaging with the finger stop or back stop. These seating lugs are moved elastically as the backstop engages with the shoulders, causing the shoulders to move over the peripheral edges of the backstop, deform elastically and then return to their initial undeformed state as the edge of the backstop is seated.

According to yet another object, the one or more extending walls, prehensile shoulders and curled lips are closed by a rear covering extending from from a rear edge of at least one extending wall to a rear edge of the other extending wall. In such a configuration, the endpoint injection signalling device is essentially closed all the way around, to prevent, for example, tampering of the device by the user, and or accidental ingress of fluids, dust, etc, which might potentially interfere with the functioning of the NFC circuit.

According to another object, the rear covering comprises a rotatable hinge, for example, to facilitate mounting of the injection endpoint signalling device on the pre-filled syringe, and then subsequent closure of the rear covering once the device has been mounted on pre-filled syringe.

Accordingly, one further object foresees that the rotatable hinge is provided along an edge of one of the extending walls. In this manner, the rear cover is thus essentially configured as a panel or door, having a hinged articulation which is aligned with one of the edges of one of the extending walls. The rear cover can also be provided with a corresponding opposing latch mechanism, and a corresponding opposing recess provided in the opposite edge of the opposite wall, to receive the latch, to secure the rear cover when moved from an open position during mounting of the signalling device to a closed position after mounting on the pre-filled syringe.

Briefly, the injection end point signalling device is designed to function as follows:
The injection endpoint signalling device is mounted onto an outside surface of a pre-filled syringe having an axially translatable needle shroud, which translates the shroud along a central longitudinal axis of the pre-filled syringe from a first, shroud retracted position before an injection, to a second, shroud extended position on completion of an injection, in which second position the needle of the pre-filled syringe is completely covered, thereby rendering the pre-filled syringe safe for subsequent disposal by the user. The signalling device has an activation switch which is positioned in parallel alignment to the central longitudinal axis of the pre-filled syringe, and which switch is furthermore positioned in a recess provided near the proximal end of the shroud. The proximal recess of the shroud receives a projecting spur that is provided on an abutment sleeve in fixed contact with, and located near, the distal end of the syringe barrel. When injection is complete, the plunger head of the pre-filled syringe activates the release mechanism for the shroud, and the shroud is moved in a distal direction along the central longitudinal axis. The relative movement of the shroud compared to the fixed position of the abutment sleeve causes the proximal recess to move in a proximal direction as part of the shroud, until it comes into contact with the projecting spur of the abutment sleeve. At this point, the projecting spur enters the recess, and engages with the activation switch, causing it to be moved from the "off" position to the "on" position. Given that the projecting spur prevents any further axial movement of the shroud, the activation switch remains in the "on" or active state in this position, and the injection endpoint information, be it a separately stored data bit, an electrical impulse, or simply the detection of current flow, is made accessible to the NFC circuit, which can be energized in the known manner, for example, by approaching the pre-filled syringe to an NFC equipped smartphone or a corresponding NFC reader, or vice-versa. The suitably energized NFC circuit of the endpoint signalling device can then cause any tag information stored in the NFC circuit, including the recently rendered accessible endpoint information, to be broadcast, and/or communicated, and/or received by the reader, in the known manner and functioning of NFC circuitry.

### BRIEF DESCRIPTION OF THE FIGURES

The invention will now further be described in relation to the figures, provided for illustrative purposes of various embodiments of the invention:
Figures 1A and 1B represent schematic perspective views of a top and bottom of a pre-filled syringe equipped with a passive needle safety shroud mechanism, on which the endpoint signalling device according to the invention has been mounted;
Figures 2A and 2B represent schematic axial views of the front and rear of the prefilled syringe of Figure 1 equipped with the endpoint signalling device according to the invention;
Figures 3A and 3B represent schematic perspective views of the endpoint signalling device according to the invention, seen from underneath, showing a detail relating to the location of the near field communications circuit;
Figures 4A and 4B represent schematic perspective views of further details of the endpoint signalling device according to the invention;
Figures 5A and 5B represents schematic cross-sectional views of a pre-filled syringe with a passive needle safety shroud mechanism as shown in Figure 1, showing the two main positions of the needle shroud and corresponding endpoint signalling device according to the invention.

### EXAMPLE

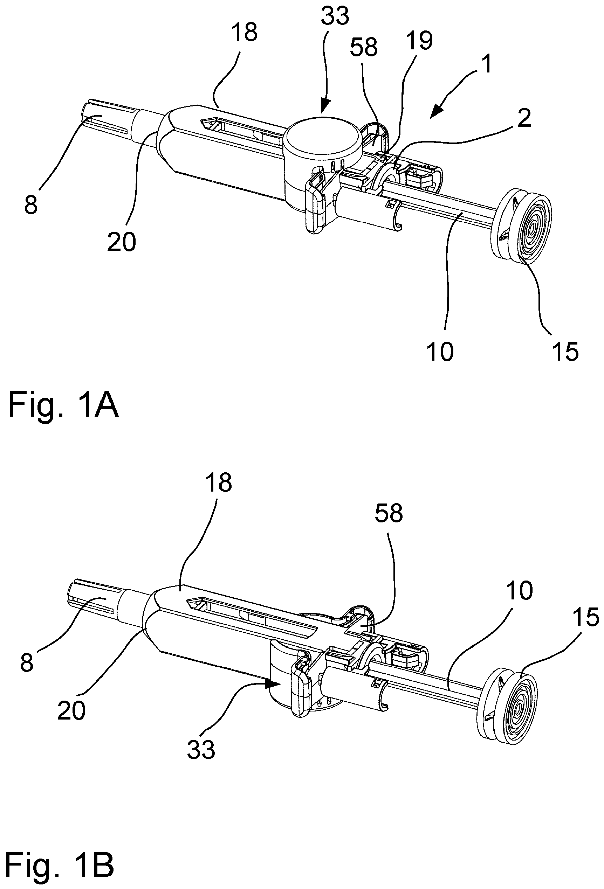

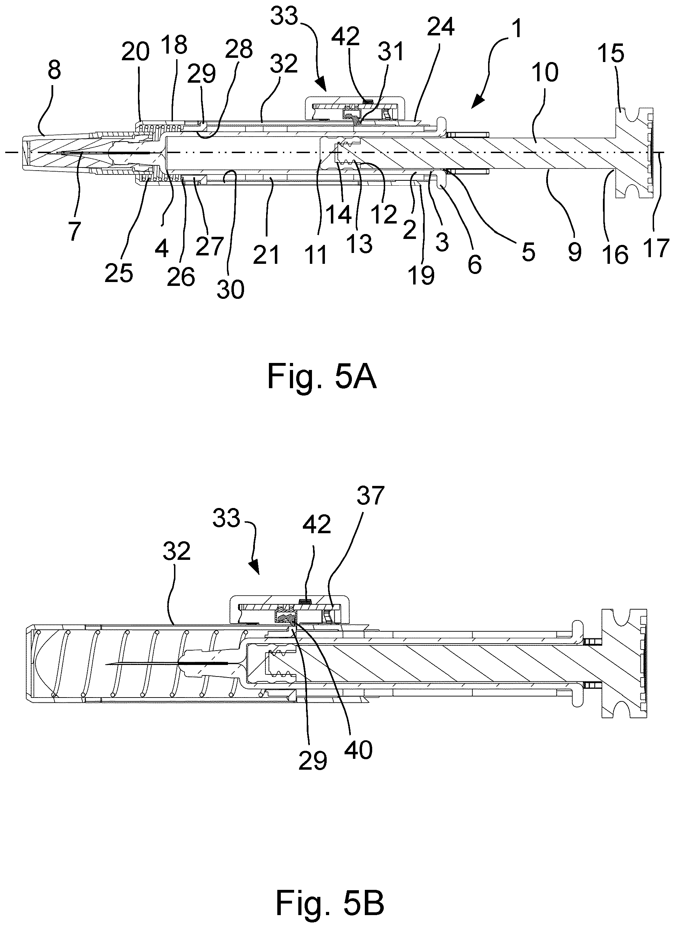

Turning now to the figures, a safety-shroud equipped pre-filled syringe (1) is illustrated in perspective top and bottom views respectively in Figures 1A and 1B, and in more detail in the illustrative cross-sectional representations of Figures 5A and 5B. The pre-filled syringe (1) has an elongated hollow syringe body (2) having a proximal extremity (3) and a distal extremity (4), with a first opening (5) at the proximal extremity (3) and a collar (6), or flange, projecting outwardly of the hollow syringe body (2) at said proximal extremity (3) around said first opening (5). An injection needle (7) covered by a removable or frangible needle cap (8) is mounted at the distal extremity (4) of the hollow elongated syringe body (2) and closes a second, distal opening (7) of the hollow elongated syringe body (2) at said distal extremity (4). A controlled amount of injectable material (not shown) such as a drug in liquid or form, is introduced into the hollow body (2) during assembly of the syringe components.

A plunger (9) is configured and dimensioned to be inserted into the hollow elongated syringe body (2) via the proximal extremity (3) and corresponding proximal opening (5) of the hollow syringe body (2), the plunger (9) having a plunger body or rod (10) comprising a stopper (11) located at a distal extremity (12) of the plunger body (10). The stopper (11) can be connected in a known way to the plunger body (10), for example, through the provision of a screw threaded projection (13) at the distal extremity (12) of the plunger body (9), and a corresponding screw-threaded bore (14) provided inside the stopper (11). The plunger body (10) further has a plunger head (15) located at a proximal extremity (16) of said plunger body (10). The plunger (9) and syringe body (2) are in substantial longitudinal alignment along a central longitudinal axis (17) of the syringe body (2). A needle shroud (18) extends along and around the outside of the syringe body (2) and has a proximal extremity (19) and a distal extremity (20). A commercial product with a safety needle shroud such as the one illustrated is sold under the tradename BD Ultrasafe Passive ^{™}, by Becton Dickinson.

As can be seen in greater detail in Figures 5A and 5B, the needle shroud (18) is configured to move from a first, shroud retracted position (cf. Figure 5A), in which, once the frangible or removable needle cap (8) has been removed, the needle (7) is exposed, to a second, shroud extended position, as illustrated in Figure 5B, which is effective only on completion of an injection, and in which the needle (7) is completely covered by the needle shroud (18). The needle shroud (18) defines a bore (21) and has a proximal end (19) and a distal extend (20). The shroud (18) extends from the proximal end (19), located adjacent to the proximal end (3) of the syringe (2), along the outside of the syringe (2), over and distally beyond the distal end (4) of the syringe (2) to the distal end (20) of the shroud (18), such that the syringe (2) is held within the bore (21) of the shroud (18). The shroud (18) engages against the syringe (2) at the proximal end (19) of the shroud via an elastically deformable portion (24). A compressed biasing spring (25) is located within the bore and seated against the distal end (20) of the shroud (18). A proximal end (26) of spring (25) abuts against an abutment sleeve (27) which surrounds, and is seated in a fixed position on, an outer surface (28) of the syringe (2), proximally from, but adjacent, to the proximal end (4) of the syringe (2). The abutment sleeve (27) further comprises at least one projecting spur (29), which engages in sliding engagement with an inward facing surface (30) of the shroud, for example a groove which is longitudinally aligned with the central longitudinal axis (17). The shroud (18) is further provided with a recess (31) or orifice, that extends from the outward facing surface (32) of the shroud (18) to the inward facing surface (30) of the shroud (18), the recess or orifice (31) being located distally from, but adjacent to, the proximal end (19) of the shroud (18).

As illustrated in greater detail in Figures 5A and 5B, an injection endpoint signalling device (33) is mounted on, and engages with the outward facing surface (32) of the shroud (18). In the initial, shroud-retracted position (Figure 5A), the injection endpoint signalling device (33) is located near the proximal end (19) of the shroud (18), as will be described in more detail hereafter.

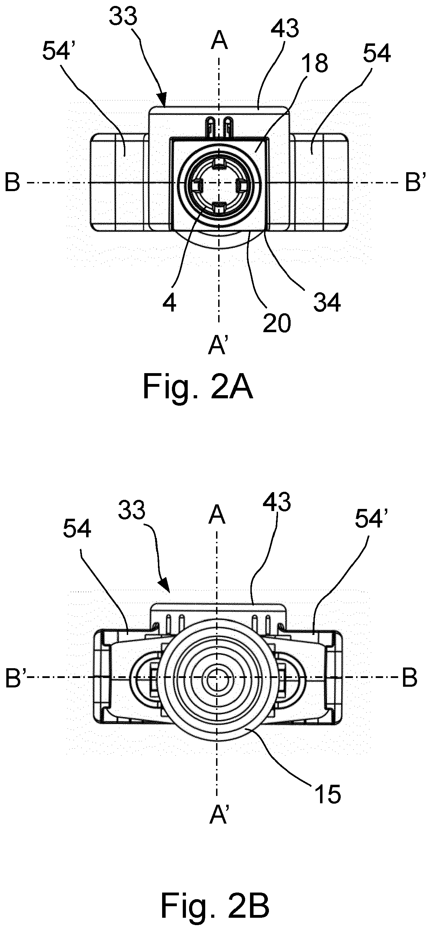

Figures 2A and 2B illustrate schematically the view of the injection endpoint signalling device (33) when mounted on the pre-filled syringe equipped with needle safety shroud. Figure 2A shows the view along the central longitudinal axis (17) of pre-filled syringe the from the distal ends (4, 20), and Figure 2B the view from the proximal ends (3, 19) of the pre-filled syringe equipped with the needle safety shroud. As will be apparent from these views, taken in conjunction with Figures 1A and 1B, it can be seen that the injection endpoint signalling device (33) spans the width of the shroud (18), and lies in a plane that is both orthogonal (A-A') and parallel (B-B') to the central longitudinal axis, and additionally engages with a respective sidewall (34) of the shroud in parallel to said central longitudinal axis. As can be seen from Figures 2A and 2B, the injection endpoint signalling device (33) resembles a button cap positioned on and across the width of the shroud (18), slightly raised from the outward facing surface (32) thereof.

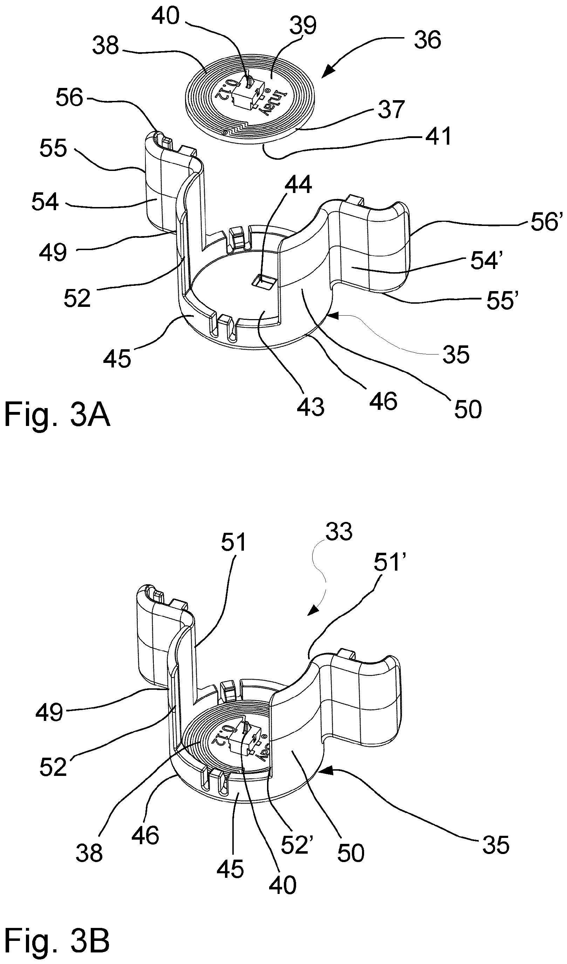

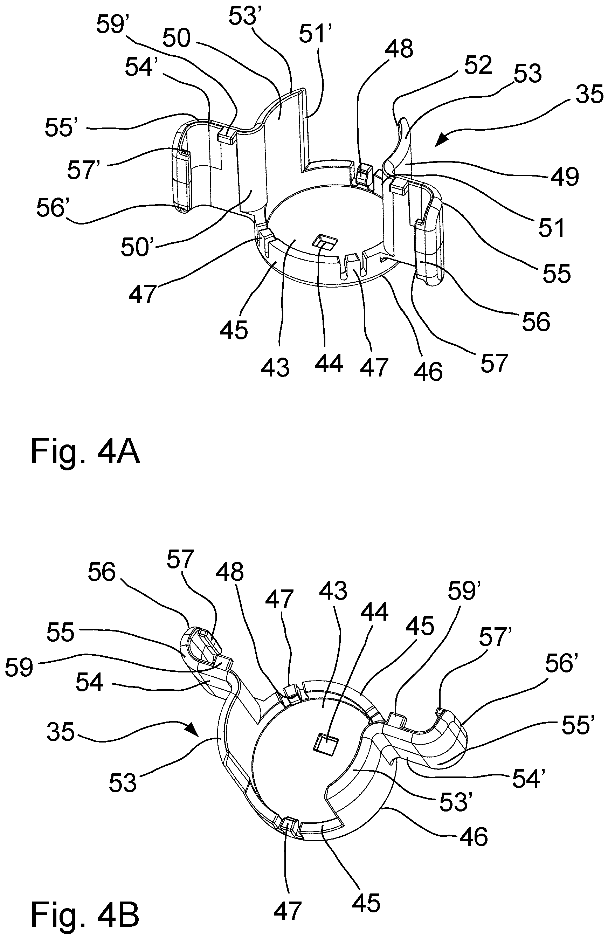

Figures 3A, 3B, 4A and 4B represent more detailed illustrative views of the injection endpoint signalling device (33), in particular perspective views of the relative component parts of the injection endpoint signalling device (33).

Figures 3A and 3B show a circuit holder body (35), in a rear perspective view to expose further details of the injection endpoint signalling device (33). The circuit holder body (35) is shaped and configured to receive and retain a near field communications (NFC) circuit (36), the type and functioning of which are known per se. The NFC circuit (36) comprises a disk-shaped printed circuit board (37), and integrates an antenna (38) on a first face (39) of the disk-shaped circuit board (37), and an activation switch (40). On the opposite, second face (41) of the circuit board (37), a NFC micro-controller is provided (42, Fig. 5A, 5B) for controlling the functioning of the NFC circuit (36). The circuit holder body (35) comprises a disk-shaped, or substantially disk-shaped, base (43), which is configured and dimensioned to receive and retain the disk-shaped circuit board (37). To that end, the base (43) of the circuit holder body (37) is provided with a socket (44) that is dimensioned and positioned to receive and seat the NFC micro-controller (42) located on the second, opposite face (41) of the circuit board (37). The socket (44) further enables the activation switch (40) on the opposite face (41) of the circuit board (37) to be correctly positioned in parallel alignment to the central longitudinal axis (17) of the pre-filled syringe (1), when the endpoint signalling device (33) is mounted on the shroud (18). As can be seen from Figures 1A and 1B, when the endpoint signalling device (33) is mounted on the shroud (18), and the shroud (18) is in the initial, retracted position, the activation switch (40) penetrates from the outside surface (32) and is lodged within, the recess (31) to extend into the bore (21) of the shroud (18). The base (43) is also provided with a peripheral wall (45) extending from and around the periphery (46) of the base (43), the peripheral wall (45) being provided with one or more radially spaced apart retaining lugs (47), which include a head portion (48) that projects into the inner volume defined by the base (43) and peripheral wall (46). As the circuit board (37) is inserted into this inner volume, the lugs (47) deform elastically radially outwardly, to allow passage of the disk-shaped circuit board, then move back in again to close over the circuit board, with the projecting head portions (48) engaging in retaining surface engagement with the first face (39) of the circuit board (37).

As illustrated in Figures 3A, 3B, 4A and 4B, the circuit holder body (35) further comprises a pair of elastically deformable side walls (49, 50) extending in the same direction from the periphery of the base (43), and orthogonally to said base (43). The side walls (49, 50) each have a first end (51, 51') and a second end (52, 52'), and an outside edge (53, 53'). The side walls (49, 50) have an arcuate shape, corresponding to the arc defined by the periphery of the disk-shaped base (43), and also extend at least partly around the periphery of the base (43), with the respective first (51, 51') and second (52, 52') ends defining a space there-between that is slightly smaller than the width of the shroud (18), such that on mounting of the endpoint signalling device (33), the side walls deform elastically and engage frictionally and elastically, via their respective first (51, 51') and second ends (52, 52'), with corresponding side walls (34, 34') of the shroud (18), either side of the central longitudinal axis (17). The circuit holder body (35) further comprises a pair of prehensile shoulders, each shoulder (54, 54') extending away from and substantially orthogonal to the central longitudinal axis (17) from the first, or proximal, end (51, 51') of each of the respective extending side walls (49, 50). The prehensile shoulders (54, 54') extend from a first, radially distant end (55, 55') of the shoulder (54, 54') to a second end (56, 56') spaced apart from the first end of the shoulder, to form a curled lip (57, 57'), configured to engage in elastically deformable clasping engagement with a corresponding finger stop or backstop (58) which extends orthogonally outwardly from the body (2) of the pre-filled syringe (1), and which is mounted on the shroud (18). The prehensile shoulders (54, 54') are advantageously provided with one or more elastically deformable seating lugs (59, 59'), which extend away from the shoulder, to assist in engaging with the finger stop or back stop. These seating lugs (59, 59') are moved elastically as the backstop (58) engages with the shoulders during mounting of the endpoint signalling device (33), causing the shoulders (54, 54') to move over the peripheral edges of the backstop (58), deform elastically and then return to their initial undeformed state as the edge of the backstop (58) is seated on the shoulders (54, 54').

As is visible from the figures, in particular from Figure 1B, the circuit holder body (35) is represented as backless, i.e. it has no rear cover. However, and whilst not illustrated, it can be useful to provide the one ore more of the extending walls, prehensile shoulders and curled lips with a rear closure covering extending from from a first edge (53) of at least one extending side wall (49) to an opposite facing edge (53') of the other extending side wall (50). The rear covering can further be provided with a rotatable hinge, located along, for example, one of the edges (53, 53') of one of the extending side walls (49, 50). This is particularly advantageous for example, to prevent some ingress of dust and or liquids into the endpoint signalling device (33), but particularly to prevent tampering by a user of any of the components of the endpoint signalling device, for example, the circuit board, antenna, NFC micro-controller and/or activation switch. Such an articulated rear covering would of course be open when mounting the endpoint signalling device (33) onto the shroud (18), and then closed shut once mounting of the device (33) had been completed. The closure of the rear covering can be suitably provided via a combination of a latch on the rear covering, and a corresponding receiving recess for the latch provided on the edge (53') opposite the edge (53) on which the articulation or hinge point is provided.

Turning once again to Figures 5A and 5B, the functioning of the endpoint signalling device (33) will now be explained. In the shroud retracted position illustrated in Figure 1A, the shroud (18), which is the position adopted by the shroud before, and during injection, the shroud (18) exposes the needle (7) once the frangible or detachable needle cap (8) has been removed. The activation switch of the endpoint signalling device lies in parallel to the central longitudinal axis (17) and is engaged in the recess (31) of the shroud (18), and extends into the bore (21) thereof. As injection proceeds, the plunger (9) and plunger head (15) are moved in a distal direction towards the proximal end (3) of the syringe. When injection is completed, the plunger (9) and plunger head are located at the proximal end of the syringe, and the stopper (11) of the plunger (9) is located at the distal end (4) of the syringe. At this point, the needle safety mechanism is activated, for example, as described elsewhere in the present specification, causing the compressed biasing spring to expand and push against the abutment sleeve (27), which is in fixed positional contact with the outside surface of the syringe body (2). The abutment sleeve (27) and syringe body (2) are moved in a direction opposite to that of the shroud (18), which is moved from the retracted position into the extended position covering the needle. The relative opposing translational movements along the central longitudinal axis (17) of the shroud (18) with respect to the syringe body (2) are arrested when the abutment sleeve (31) and associated projecting spur (29) have moved along the inside surface of the shroud (18) to the point where the projecting spur (29) engages in the recess (31). It is at this point that the projecting spur (29) also comes into surface contact with the activation switch (40). In the example illustrated in the Figures, the switch is moved up, and out, of the recess (31) as the projecting spur enters the recess, thereby moving the switch from the inactive, or "off" state to the active, or "on" state. The moving of the activation switch from the "off" to the "on" state renders an injection endpoint information which was previously inaccessible to the NFC circuit, visible or accessible to said NFC circuit. The syringe (1) can now be brought near to a NFC equipped device such as a smartphone or NFC reader, which will energize the NFC circuit (36) within the endpoint signalling device (33) and cause any information stored in the NFC circuit, including the now accessible injection endpoint information, to be communicated to the NFC reader or NFC-equipped smartphone device.

## Claims

1. Pre-filled syringe comprising a post-injection needle shroud, the needle shroud being configured to translate from a first position in which the shroud is retracted and the needle of the pre-filled syringe is exposed, to a second position in which the shroud is extended and the needle of the pre-filled syringe is completely surrounded by the shroud, and an injection endpoint signalling device mounted onto the pre-filled syringe,
wherein:
the injection endpoint signalling device comprises a wireless injection endpoint signalling system comprising a near field communications (NFC) circuit and an activation switch; and
in the first, shroud-retracted position, the activation switch maintains the NFC circuit in an inactive state in which said NFC circuit is off, and in which an injection endpoint information is inaccessible to the NFC circuit; and
in the second, shroud-extended position, the activation switch maintains the NFC circuit in an active state in which said NFC circuit is on, and in which the injection endpoint information is accessible to the NFC circuit;
wherein the activation switch is moved from the inactive state to the active state via mutually cooperating surface engagement between a part of the shroud and the activation switch.

2. Pre-filled syringe according to claim 1, wherein the mutually cooperating surface engagement between the activation switch and the needle shroud is provided when the shroud is positioned in a fully extended position.

3. Pre-filled syringe according to claim 1 or claim 2, wherein the injection endpoint signalling device comprises a near field communications circuit holder body, and the circuit holder body is mounted on an outward facing surface of a longitudinal body of the pre-filled syringe.

4. Pre-filled syringe according to claim 3, wherein the circuit holder body is mounted on an outward facing surface of the needle safety shroud.

5. Pre-filled syringe according to any one of claims 3 or 4, wherein the circuit holder body is mounted on the pre-filled syringe in a plane that lies parallel to the central longitudinal axis.

6. Pre-filled syringe according to any one of claims 3 to 5, wherein the circuit holder body comprises a socket configured and dimensioned to receive and locate a microcontroller of the near field communications circuit.

7. Pre-filled syringe according to any one of claims 3 to 6, wherein the near field communications circuit is integrated into a disk-shaped circuit board, the microcontroller is located on a first face of the circuit board, and the activation switch is located on an opposite, second face of the circuit board.

8. Pre-filled syringe according to any one of claims 3 to 7, wherein the first face of the disk-shaped circuit board is held against an inward facing surface of a disk-shaped base of the circuit holder body by at least one or more retaining lugs.

9. Pre-filled syringe according to claim 8, wherein the retaining lugs are radially distributed about an axis of rotation of the disk-shaped base of the circuit holder body.

10. Pre-filled syringe according to claim 9, wherein the axis of rotation of the disk-shaped base lies perpendicular to the central longitudinal axis of the pre-filled syringe.

11. Pre-filled syringe according to claim 9 or claim 10, wherein the axis of rotation of the disk-shaped base lies perpendicular to the horizontal plane which is parallel to the central longitudinal axis.

12. Pre-filled syringe according to any one of claims 3 to 11, wherein the circuit holder body comprises at least one or more walls located on a periphery of, and extending in the same direction away from, the disk shaped base.

13. Pre-filled syringe according to claim 12, wherein the at least one or more extending walls are arc shaped and, when the device is mounted on the pre-filled syringe and/or the needle safety shroud, said walls engage in elastically deformable abutment with at least one side wall of the pre-filled syringe and/or the needle safety shroud.

14. Pre-filled syringe according to claim 12 or claim 13, wherein the at least one or more extending walls each comprise a prehensile shoulder, extending substantially orthogonal to the central longitudinal axis from a proximal end of each of the respective extending walls.

15. Pre-filled syringe according to claim 14, wherein the prehensile shoulder extends, from a radially distant end of the shoulder, in a proximal direction to form a curled lip, configured to engage in elastically deformable clasping engagement with a corresponding finger stop which extends orthogonally outwardly from the body of the pre-filled syringe.

16. Pre-filled syringe according to any one of claims 12 to 15, wherein one ore more of the extending walls, prehensile shoulder and curled lip are closed by a rear covering extending from from a rear edge of at least one extending wall to a rear edge of the other extending wall.

17. Pre-filled syringe according to claim 16, wherein the rear covering comprises a rotatable hinge.

18. Pre-filled syringe according to claim 17, wherein the rotatable hinge is provided along an edge of one of the extending walls.

19. Pre-filled syringe according to any one of claims 1 to 18, wherein the circuit board activation switch, when the circuit holder body is mounted on the pre-filled syringe body and/or the needle safety shroud, is located in parallel to, and along, the central longitudinal axis.

20. Pre-filled syringe according to claim 1, wherein the activation switch is moved from the inactive state to the active by cooperating surface engagement between a proximal part of the shroud and the activation switch.

21. Pre-filled syringe according to claim 1, wherein the activation switch is a displaceable, or movable, electrical contact.

22. Pre-filled syringe according to claim 21, wherein the displaceable, or movable, electrical contact is selected from the group consisting of a microswitch, a biased, or constrained, electrically conducting metal strip, and a movable electrically conducting surface.
